# EUROPEAN PATENT APPLICATION

(11) **EP 4 631 932 A1**
(43) Date of publication of application: **15.10.2025**
(21) Application number: 23899854.6
(22) Date of filing: 29.11.2023
(51) Int. Cl.: C07D 213/75, C07D 513/04

(54) **PREPARATION METHOD FOR EDOXABAN AND INTERMEDIATE THEREOF**

(30) Priority: 05.12.2022 CN 202211546053
(71) Applicant: Zhejiang Huahai Pharmaceutical Co., Ltd., Taizhou, Zhejiang 317024 (CN); Shanghai Syncores Technologies, Inc., Shanghai 201203 (CN)
(72) Inventor: MOU, Peng, Shanghai 201203 (CN); ZHAI, Qisong, Shanghai 201203 (CN); ZHANG, Yu, Shanghai 201203 (CN); HUANG, Luning, Shanghai 201203 (CN); YANG, Zhengyi, Shanghai 201203 (CN); TAO, Anping, Shanghai 201203 (CN); GU, Hong, Shanghai 201203 (CN)
(74) Representative: v. Bezold & Partner Patentanwälte - PartG mbB
(86) International application number: PCT/CN2023/135077
(87) International publication number: WO 2024/120275

(57) **Abstract**

The present invention provides a preparation method for Edoxaban or a salt or hydrate thereof. The method comprises the following steps: step 1: under acidic conditions and in a polar solvent, removing the N-Boc protecting group of chemical compound 1 so as to generate chemical compound 2; step 2: adding to the reaction solution obtained in step 1 triethylamine, a protic solvent, a condensing agent, and 4,5,6,7-tetrahydro-5-methyl-thiazolo[5,4-C]pyridine-2-carboxylic acid or a salt thereof, and reacting to obtain Edoxaban. Adding a certain amount of protic solvent during the reaction accelerates the reaction process, shortens the reaction time, improves reaction efficiency, and reduces the impurity content in the obtained Edoxaban. The purification method is simple and convenient, produces high purity, and is suited for large-scale industrial production.

## Description

### FIELD OF THE INVENTION

The present application relates to the field of chemical synthesis, particularly to a preparation method for edoxaban and intermediates thereof.

### BACKGROUND OF THE INVENTION

Edoxaban, developed by Daiichi Sankyo Co., Ltd., Japan, is an oral blood coagulation factor X (FXa) inhibitor. It was approved by U.S. FDA in 2015 for the reduction of the risk of stroke and systemic embolism in patients with non-valvular atrial fibrillation (atrial fibrillation, AF). Edoxaban is also approved for patients with deep venous thrombosis and pulmonary embolism who have been administered with parenteral anticoagulants for 5 to 10 days. Edoxaban became the fourth novel oral anticoagulant (NOAC) approved by FDA, followed by Dabigatran, Rivaroxaban, and Apixaban.

The chemical name of edoxaban is N-(5-chloropyridin-2-yl)-N'-[(1S,2R,4S)-4-(N,N-dimethylcarbamoyl)-2-(5-methyl-4,5,6,7-tetrah ydro[1,3]thiazolo[5,4-c]pyridine-2-carboxamido)cyclohexyl]oxamide, and the structural formula thereof is shown as follows:

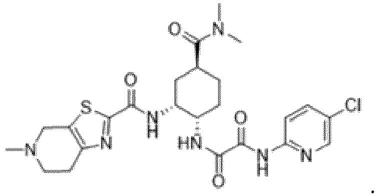

In recent years, the incidence of cardiovascular diseases is on the rise, and the demand of patients for anticoagulants is increasing. Anti-blood coagulant factor Xa agents have become a main development trend of anticoagulants. Therefore, edoxaban has a very broad commercial prospect.

Patent No. CN1826333 of Daiichi Sankyo Co., Ltd. discloses a synthetic route of edoxaban, in which the compound of formula 1 is a key intermediate. Reference Example 434 discloses its preparation method for the first time, in which methyl [(5-chloropyridin-2-yl)amino]-2-oxoacetate hydrochloride is dissolved in tetrahydrofuran, converted to a lithium salt using an aqueous solution of lithium hydroxide, and then reacted with tert-butyl {(1R,2S,5S)-2-amino-5-[(dimethylamino)carbonyl]cyclohexyl}carbamate oxalate in the presence of HOBt and EDC as a condensing agent to obtain edoxaban intermediate compound 1. The edoxaban intermediate compound 1 is hydrolysed to remove the Boc protecting group and then condensed with 5-methyl-4,5,6,7-tetrahydro[1,3]thiazolo[5,4-c]pyridine-2-carboxylic acid or a salt thereof to obtain edoxaban: 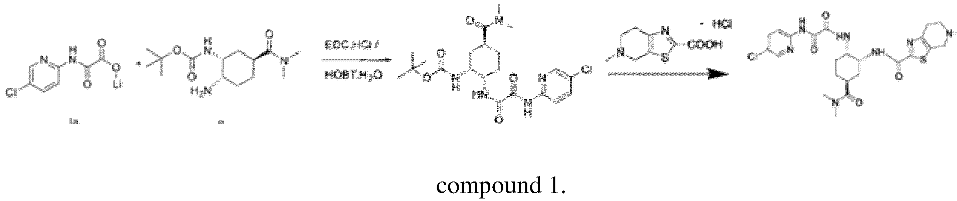 This method requires purification by silica gel column chromatography, making it unsuitable for large-scale industrial production.

Chinese Patent No. CN103214414 discloses a preparation method for edoxaban, in which triethylamine is added to a suspension of tert-butyl {(1R,2S,SS)-2-amino-5-[(dimethylamino)carbonyl]cyclohexyl}carbamate oxalate in acetonitrile at 60°C, then ethyl [(5-chloropyridin-2-yl)amino]-2-oxoacetate hydrochloride is added at the same temperature. After stirring for 6 hours, the mixture is further stirred at room temperature for 16 hours. At the end, water is added to the reaction solution, which is cooled to 10°C for crystallisation. The resultant is filtered to obtain edoxaban intermediate compound 1. Then methanesulfonic acid is added to the solution of edoxaban intermediate compound 1 in acetonitrile for deprotection. After that, triethylamine, EDCI, HOBt and 5-methyl-4,5,6,7-tetrahydro-thiazolo[5,4-c]pyridine-2-carboxylic acid hydrochloride are added to the reaction solution. The mixture is stirred and reacted for 16 hours. After the reaction is completed, triethylamine and water are added to the resultant, and it is cooled for crystallisation to obtain edoxaban. During the step for preparing edoxaban intermediate compound 1, the reaction system solidifies instantly, becomes unstirrable, and finally results in reduced product yield and purity. Moreover, the condensation reaction after deprotection is time-consuming.

To solve the problem of solidification that occurred in the large-scale preparation of edoxaban intermediate compound (1), Chinese Patent No. CN102348688 discloses changing the feeding order and adding triethylamine in batches. In particular, pre-treating ethyl [(5-chloropyridin-2-yl)amino]-2-oxoacetate hydrochloride with a portion of triethylamine in acetonitrile, followed by adding tert-butyl {(1R,2S,5S)-2-amino-5-[(dimethylamino)carbonyl]cyclohexyl}carbamate oxalate and additional triethylamine for reaction.

Chinese Patent No. CN108484641 discloses a preparation method for edoxaban intermediate compound (1), in which 1-butyl-3-methylimidazolium hydroxide, tert-butyl {(1R,2S,5S)-2-amino-5-[(dimethylamino)carbonyl]cyclohexyl}carbamate oxalate, ethyl [(5-chloropyridin-2-yl)amino]-2-oxoacetate hydrochloride and acetonitrile are added, respectively. The mixture is stirred. Then triethylamine and pyridine are added. The turbid liquid is stirred and heated until the system is heated up to 45°C to 60°C. When the system becomes viscous and difficult to stir, acetonitrile is added in batches to appropriately adjust the system to a non-viscous state. After reacting for 4 hours, a solution of edoxaban intermediate compound 1 in acetonitrile is obtained. Then 1-butyl-3-methylimidazolium hydroxide and methanesulfonic acid are added to the acetonitrile solution, and the mixture is cooled to 10°C in an ice bath. Under stirring, triethylamine and pyridine are added, followed by EDCI, HOBt, and 5-methyl-4,5,6,7-tetrahydro-thiazolo[5,4-c]pyridine-2-carboxylic acid hydrochloride. The reaction proceeds for over 20 hours to obtain edoxaban.

Chinese Patent No. CN107641131 discloses a preparation method for edoxaban, in which tert-butyl {(1R,2S,5S)-2-amino-5-[(dimethylamino)carbonyl]cyclohexyl}carbamate oxalate is reacted with ethyl [(5-chloropyridin-2-yl)amino]-2-oxoacetate hydrochloride in DMF, using sodium acetate as a base, and the reaction is heated to 90°C to 110°C for 2 to 3 hours to obtain compound 1; then the Boc protecting group of compound 1 is removed in a solution of HCl in ethanol to obtain compound 2; 5-methyl-4,5,6,7-tetrahydro-thiazolo[5,4-c]pyridine-2-carboxylic acid hydrochloride is condensed in a polar solvent in the presence of a base and CDI as a condensing agent to form compound 3, which is then reacted with compound 2 to generate compound 4, wherein the reaction of 5-methyl-4,5,6,7-tetrahydro-thiazolo[5,4-c]pyridine-2-carboxylic acid hydrochloride with the condensing agent CDI requires 7 hours, and the subsequent reaction with compound 2 requires 20 hours;

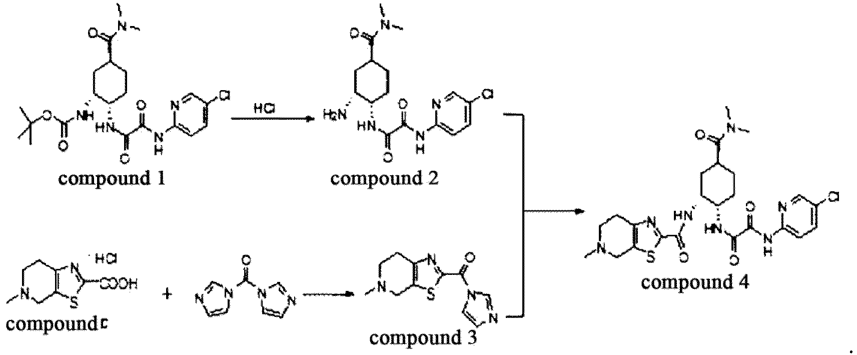

The preparation methods for edoxaban in the prior art generally involve a reaction between ethyl [(5-chloropyridin-2-yl)amino]-2-oxoacetate or a hydrochloride salt thereof and tert-butyl {(1R,2S,5S)-2-amino-5-[(dimethylamino)carbonyl]cyclohexyl}carbamate oxalate to obtain compound 1, and a reaction between deprotected compound 1 and a condensation substance of 5-methyl-4,5,6,7-tetrahydro-thiazolo[5,4-c]pyridine-2-carboxylic acid hydrochloride. These reactions are time-consuming, with high impurity content, and require large amounts of base for neutralization and participation in the reaction due to the poor stability of ethyl [(5-chloropyridin-2-yl)amino]-2-oxoacetate hydrochloride. Moreover, there are problems such as viscosity and solidification of the reaction system or the need for large amounts of solvent to dilute the reaction mixture to prevent solidification. Therefore, it is highly necessary to provide a simple and low-cost edoxaban preparation method for large-scale industrial production.

### SUMMARY

In view of the disadvantages and deficiencies in the prior art, the present application provides a preparation method for edoxaban, in which a protic solvent is added during the condensation reaction to accelerate the reaction, which shortens the reaction time, improves the reaction efficiency, reduces the impurities in the product, and improves the product quality. Furthermore, the problem of viscosity and solidification of the reaction system can be solved by specific reaction steps effectively, eliminating the need for batchwise addition of reagents. The production operations are simple, and the technical solution of the present application is suitable for industrial production.

The present application provides a preparation method for edoxaban or a salt or hydrate thereof, comprising following steps:
step 1: under acidic condition and in a polar solvent, removing a N-Boc protecting group of compound 1 to generate compound 2; 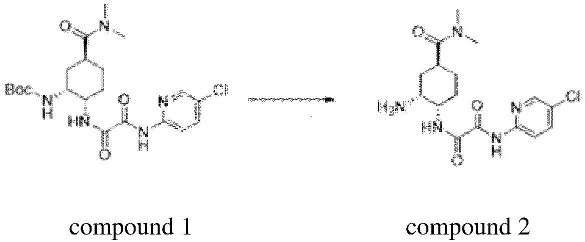
step 2: adding triethylamine, a protic solvent, a condensing agent, and 5-methyl-4,5,6,7-tetrahydro-thiazolo[5,4-c]pyridine-2-carboxylic acid or a salt thereof into a reaction solution obtained in step 1, and reacting to obtain edoxaban;

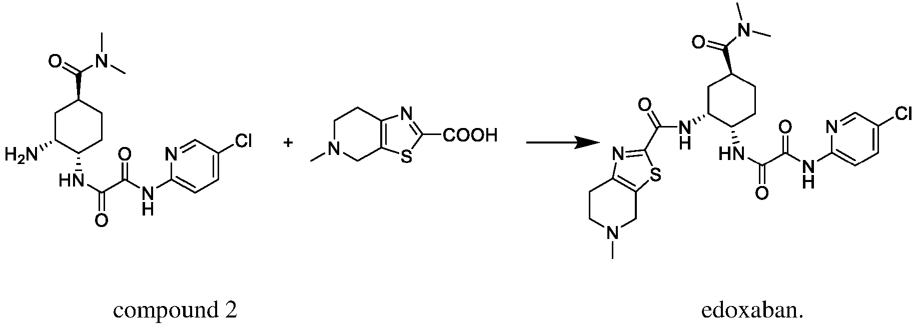

In some embodiments, the acidic condition in step 1 is provided by hydrochloric acid, trifluoroacetic acid, or methanesulfonic acid, preferably methanesulfonic acid.

In some embodiments, the polar solvent is selected from the group consisting of methanol, ethanol, propanol, acetonitrile, and a combination thereof, preferably acetonitrile.

In some embodiments, the protic solvent in step 2 is selected from the group consisting of methanol, ethanol, water, and a combination thereof.

In some embodiments, the protic solvent is in a usage amount of 0.5 wt.% to 5 wt.% of the total weight of all materials in a reaction system of step 1; wherein the all materials in the reaction system of step 1 refer to the total mass of compound 1, the acidic substance, and the polar solvent.

In some embodiments, the protic solvent is in a usage amount of 3.5 wt.% to 5 wt.% of the total weight of all materials in the reaction system of step 1.

In some embodiments, the condensing agent is a combination of EDCI and HOBt.

In some embodiments, the compound 1, the acid used for the acidic condition, and the polar solvent are in a mass ratio of 1:0.6:15 to 1:0.9:20.

In some embodiments, the compound 1, the 5-methyl-4,5,6,7-tetrahydro-thiazolo[5,4-c]pyridine-2-carboxylic acid or the salt thereof, and the triethylamine in step 2 are in a molar ratio of 1:1.1:6 to 1:1.2:8.

In some embodiments, the compound 1, EDCI, and HOBt are in a mass ratio of 1:0.4:0.3 to 1:0.6:0.5.

In some embodiments, the reaction solution obtained in step 1 is treated first with the triethylamine at -5°C to 5°C, and then the protic solvent, the condensing agent, and the 5-methyl-4,5,6,7-tetrahydro-thiazolo[5,4-c]pyridine-2-carboxylic acid or the salt thereof are added.

In some embodiments, a reaction in step 2 is at a temperature of 30°C to 50°C; and the reaction in step 2 is for a time of 6 to 8 hours.

In some embodiments, a post-treatment method for the edoxaban obtained in step 2 is adding dropwise an aqueous solution of sodium bicarbonate which is at a temperature of 0°C to 20°C to a reaction solution obtained in step 2, controlling temperature and stirring for crystallization.

In this step, the inventor surprisingly found that adding a small amount of protic solvent to the reaction system can accelerate the reaction and reduce impurity formation.

In some embodiments, the compound 1 is prepared by following steps:
step A: adding separately methyl [(5-chloropyridin-2-yl)amino]-2-oxoacetate and tert-butyl {(1R,2S,SS)-2-amino-5-[(dimethylamino)carbonyl]cyclohexyl}carbamate oxalate to a polar solvent to obtain a reaction mixture;
step B: heating the reaction mixture obtained in step A to 60 ± 5°C, then adding triethylamine in one batch and reacting to prepare compound 1;

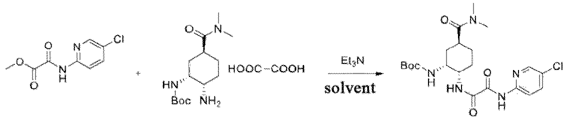

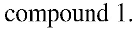

In some embodiments, the polar solvent is selected from the group consisting of methanol, ethanol, propanol, acetonitrile, and a combination thereof, preferably acetonitrile.

In some embodiments, the tert-butyl {(1R,2S,SS)-2-amino-5-[(dimethylamino)carbonyl]cyclohexyl}carbamate oxalate, the methyl [(5-chloropyridin-2-yl)amino]-2-oxoacetate, and the triethylamine in step B are in a molar ratio of 1.0:1.0:2.8 to 1.0:1.1:3.5.

In some embodiments, the tert-butyl {(1R,2S,SS)-2-amino-5-[(dimethylamino)carbonyl]cyclohexyl}carbamate oxalate and the polar solvent is in a mass-to-volume ratio of 1.0:4.5 to 1.0:5.5 g/mL.

In some embodiments, the methyl [(5-chloropyridin-2-yl)amino]-2-oxoacetate is prepared by following steps:
(1) reacting 2-amino-5-chloropyridine with methyl oxalyl chloride in tetrahydrofuran to obtain methyl [(5-chloropyridin-2-yl)amino]-2-oxoacetate hydrochloride;
(2) adding the methyl [(5-chloropyridin-2-yl)amino]-2-oxoacetate hydrochloride to tetrahydrofuran, and then adding sodium bicarbonate in batches to liberate the methyl [(5-chloropyridin-2-yl)amino]-2-oxoacetate.

During research, the inventor found that the purity of methyl [(5-chloropyridin-2-yl)amino]-2-oxoacetate hydrochloride decreased from 95.28% to 90.52% after leaving it at room temperature for 13 days, and the degradation product thereof increased from 2.5% to 7.4%. Therefore, methyl [(5-chloropyridin-2-yl)amino]-2-oxoacetate hydrochloride is unstable and unsuitable for storage. However, when methyl [(5-chloropyridin-2-yl)amino]-2-oxoacetate hydrochloride is converted to a free alkali, its stability improves; and the purity is not decreased after 13 days of storage. Using a free alkali of methyl [(5-chloropyridin-2-yl)amino]-2-oxoacetate and reacting it with tert-butyl ((1R,2S,5S)-2-amino-5-(dimethylcarbamoyl)cyclohexyl)carbamate oxalate in acetonitrile, heating to 60 ± 5°C, and adding triethylamine in one batch at this temperature effectively solves the problem of viscosity and solidification of the reaction system.

Compared with the prior art, the present application has the following positive technical effects:
1. The preparation method provided by the present application comprises adding a certain amount of protic solvent during the reaction, which accelerates the reaction process, shortens the reaction time, improves reaction efficiency, and reduces the impurity content in the obtained edoxaban. The purification method is simple and convenient, produces high purity, and is suited for large-scale industrial production.
2. The specific reaction steps of the present application can effectively solve the problem of viscosity and solidification of the reaction system, making it easy to stir and fully-reacted. It improves the yield of the reaction and the stability of the starting material, thus facilitating storage.
3. The preparation method provided by the present application does not require adding triethylamine in batches, and the usage amounts of the triethylamine and the reaction solvent are small, which saves the operation time and material costs.

### DETAILED DESCRIPTION

Specific embodiments of the present application are described in further detail below, in combination with examples. The following examples are used only to illustrate the present application, but are not intended to limit the scope of the present application.

### Reference Example 1:

### tert-butyl[(1R,2S,5S)-2-({[(5-chloropyridin-2-yl)amino](oxo)acetyl}amino)-5-(dimethyl -carbamoyl)cyclohexyl]carbamate

Triethylamine (169 mL) was added to a suspension of tert-butyl {(1R,2S,SS)-2-amino-5-[(dimethylamino)carbonyl]cyclohexyl}carbamate oxalate (100.1 g) in acetonitrile (550 mL) at 60°C. Ethyl (5-chloropyridin-2-yl)amino](oxo)acetate hydrochloride (84.2 g) was added at the same temperature. After stirring for 6 hours, the reaction was continued stirring at room temperature for 16 hours. Water was added to the reaction solution. After stirring at 10°C for 1 hour and 30 minutes, the crystals were separated and filtered to obtain a white solid.

### N-(5-chloropyridin-2-yl)-N'-[(1S,2R,4S)-4-(N,N-dimethylcarbamoyl)-2-(5-methyl-4,5,6,7-tetrahydro[1,3]thiazolo[5,4-c]pyridine-2-carboxamido)cyclohexyl]oxamide (edoxaban)

316 g of acetonitrile and 20 g of tert-butyl [(1R,2S,5S)-2-({[(5-chloropyridin-2-yl)amino](oxo)acetyl}amino)-5-(dimethylcarbamoyl)cycloh exyl]carbamate were added to a reaction flask. The mixture was stirred, and the temperature was controlled at 15°C to 25°C. 16.4 g of methanesulfonic acid was added dropwise. After the addition was completed, the mixture was maintained at 15°C to 25°C and reacted for 4 to 6 hours. After the reaction was completed, the resultant was cooled. 30.2 g of triethylamine was added dropwise while controlling the temperature at -5°C to 5°C. After the addition was completed, 12 g of 5-methyl-4,5,6,7-tetrahydro-thiazolo[5,4-c]pyridine-2-carboxylic acid hydrochloride, 10.6 g of EDCI, and 7.06 g of HOBt were added successively. After the addition was completed, the temperature was raised. The mixture was maintained at 35°C to 45°C and reacted for 16 hours. After the reaction was completed, the system was distilled under reduced pressure at 35°C to 45°C to concentrate to about 1/5 of its original volume. Then 400 g of 5% aqueous solution of sodium bicarbonate was added dropwise. After the addition was completed, the mixture was maintained at 5°C to 15°C and stirred for crystallisation for 1.5 to 3.0 hours. The resultant was filtered under suction. The filter cake was washed with 40 g of water and then dried under suction to obtain an off-white solid. The resultant was dried under reduced pressure at 40°C to 50°C for 10 to 12 hours to obtain an off-white solid, with a yield of 78.9%, a purity of 95.5%, and a deprotected impurity, i.e., the above compound 2, of 2.93%.

### Reference Example 2:

The temperature was controlled at 15°C to 30°C. 39 mL of acetonitrile was added to a reaction flask, followed by 2.0 g of methyl [(5-chloropyridin-2-yl)amino]-2-oxoacetate and 0.93 g of triethylamine. After stirring for 30 minutes, 2.9 g of tert-butyl [(1R,2S,5S)-2-amino-5-(dimethylcarbamoyl)cyclohexyl]carbamate oxalate and 1.86 g of triethylamine were further added. The reaction solution was heated to 60°C. As the reaction proceeded, the reaction solution became viscous. After the reaction was completed, most of the acetonitrile was removed by concentration. 34 mL of water was added and stirred for 1 to 2 hours. The resultant was filtered under suction, and the solid was dried in a vacuum oven at 45°C to obtain edoxaban intermediate compound 1, with a yield of 91.7% and a purity of 97.4%.

### Example 1

### Preparation of methyl [(5-chloropyridin-2-yl)amino]-2-oxoacetate

Tetrahydrofuran (225 mL) and 15 g of 2-amino-5-chloropyridine were added and stirred until dissolved to a clear solution. The temperature was heated to 40°C to 50°C. 18.6 g of methyl oxalyl chloride was added dropwise to the reaction solution within 1 hour. After the addition was completed, the mixture was cooled to 0°C to 5°C and maintained for 14 to 15 hours. The resultant was filtered under suction to obtain methyl [(5-chloropyridin-2-yl)amino]-2-oxoacetate hydrochloride.

methyl [(5-chloropyridin-2-yl)amino]-2-oxoacetate hydrochloride was added to 300 mL of tetrahydrofuran, followed by 11.7 g of sodium bicarbonate. The mixture was stirred and reacted for 3 hours. The resultant was filtered under suction, and washed with tetrahydrofuran. The filtrate was further concentrated to about 1/8 of its original volume, and cooled to 10°C to 15°C. 375 mL of water was added. The mixture was stirred for 1 to 2 hours. The resultant was filtered under suction to obtain a solid, which was dried under vacuum for 24 hours to obtain methyl [(5-chloropyridin-2-yl)amino]-2-oxoacetate.

### Example 2

The temperature was controlled at 15°C to 30°C. 350 mL of acetonitrile was added to a reaction flask, and 42.0 g of methyl [(5-chloropyridin-2-yl)amino]-2-oxoacetate and 70.0 g of tert-butyl ((1R,2S,5S)-2-amino-5-(dimethylcarbamoyl)cyclohexyl)carbamate oxalate were added successively. The reaction solution was heated to 60°C. 56.6 g of triethylamine was added in one batch at this temperature. The reaction solution was a stirrable turbid liquid without becoming viscous or solidified. After the reaction was completed, the reaction solution was cooled to 10°C to 20°C. 1050 mL of water was added and stirred for 1 to 2 hours. The resultant was filtered under suction, and the solid was dried in a vacuum oven at 45°C to obtain edoxaban intermediate compound 1, with a yield of 98.2%, a purity of 99.8%, and a total impurity of 0.2%.

### Example 3

The temperature was controlled at 15°C to 30°C. 350 mL of acetonitrile was added to a reaction flask, and 42.0 g of methyl [(5-chloropyridin-2-yl)amino]-2-oxoacetate and 70.0 g of tert-butyl ((1R,2S,5S)-2-amino-5-(dimethylcarbamoyl)cyclohexyl)carbamate oxalate were added successively. The reaction solution was heated to 55°C. 56.6 g of triethylamine was added in one batch at this temperature. The reaction solution was a stirrable turbid liquid without becoming viscous or solidified. After the reaction was completed, the reaction solution was cooled to 10°C to 20°C. 1050 mL of water was added and stirred for 1 to 2 hours. The resultant was filtered under suction, and the solid was dried in a vacuum oven at 45°C to obtain edoxaban intermediate compound 1, with a yield of 98.9%, a purity of 99.7%, and a total impurity of 0.3%.

### Example 4

The temperature was controlled at 15°C to 30°C. 350 mL of acetonitrile was added to a reaction flask, and 42.0 g of methyl [(5-chloropyridin-2-yl)amino]-2-oxoacetate and 70.0 g of tert-butyl ((1R,2S,5S)-2-amino-5-(dimethylcarbamoyl)cyclohexyl)carbamate oxalate were added successively. The reaction solution was heated to 65°C. 56.6 g of triethylamine was added in one batch at this temperature. The reaction solution was a stirrable turbid liquid without becoming viscous or solidified. After the reaction was completed, the reaction solution was cooled to 10°C to 20°C. 1050 mL of water was added and stirred for 1 to 2 hours. The resultant was filtered under suction, and the solid was dried in a vacuum oven at 45°C to obtain edoxaban intermediate compound 1, with a yield of 97.8%, a purity of 99.9%, and a total impurity of 0.1%.

### Example 5: preparation method of edoxaban (with 3.5% methanol addition)

20 g of edoxaban intermediate compound 1 obtained in Example 2 was dissolved in 316 g of acetonitrile. The temperature was controlled at 15°C to 25°C. 16.4 g of methanesulfonic acid was added dropwise. After stirring for 2 to 3 hours, the reaction was completed. The resultant was cooled and the temperature was controlled at -5°C to 5°C. 30.2 g of triethylamine was added dropwise. After the addition was completed, 12.3 g of methanol, 12 g of 5-methyl-4,5,6,7-tetrahydro-thiazolo[5,4-c]pyridine-2-carboxylic acid hydrochloride, 10.6 g of EDCI, and 12.3 g of HOBt were added successively. After the addition was completed, the temperature was heated. The mixture was maintained at 35°C to 45°C and reacted for 7 hours. After the reaction was completed, the system was distilled under reduced pressure at 35°C to 45°C to concentrate to about 1/5 of its original volume. Then 400 g of 5% aqueous solution of sodium bicarbonate was added dropwise. After the addition was completed, the mixture was maintained at 5°C to 15°C and stirred for crystallisation for 1.5 to 3.0 hours. The resultant was filtered under suction. The filter cake was washed with 40 g of water and then dried under suction to obtain an off-white solid. The resultant was dried under reduced pressure at 40°C to 50°C for 10 to 12 hours to obtain an off-white solid, with a yield of 77.6%, a purity of 98.11%, and a deprotected impurity of 1.52%.

### Example 6: preparation method of edoxaban (with 5% methanol addition)

20 g of edoxaban intermediate compound 1 obtained in Example 2 was dissolved in 316 g of acetonitrile. The temperature was controlled at 15°C to 25°C. 16.4 g of methanesulfonic acid was added dropwise. After stirring for 2 to 3 hours, the reaction was completed. The resultant was cooled, and the temperature was controlled at -5°C to 5°C. 30.2 g of triethylamine was added dropwise. After the addition was completed, 17.6 g of methanol, 12 g of 5-methyl-4,5,6,7-tetrahydro-thiazolo[5,4-c]pyridine-2-carboxylic acid hydrochloride, 10.6 g of EDCI, and 12.3 g of HOBt were added successively. After the addition was completed, the temperature was heated. The mixture was maintained at 35°C to 45°C and reacted for 7 hours. After the reaction was completed, the system was distilled under reduced pressure at 35°C to 45°C to concentrate to about 1/5 of its original volume. Then 400 g of 5% aqueous solution of sodium bicarbonate was added dropwise. After the addition was completed, the mixture was maintained at 5°C to 15°C and stirred for crystallisation for 1.5 to 3.0 hours. The resultant was filtered under suction. The filter cake was washed with 40 g of water, and then dried under suction to obtain an off-white solid. The resultant was dried under reduced pressure at 40°C to 50°C for 10 to 12 hours to obtain an off-white solid, with a yield of 78.3%, a purity of 98.25%, and a deprotected impurity of 1.32%.

### Example 7: preparation method of edoxaban (with 2% water addition)

20 g of edoxaban intermediate compound 1 obtained in Example 2 was dissolved in 316 g of acetonitrile. The temperature was controlled at 15°C to 25°C. 16.4 g of methanesulfonic acid was added dropwise. After stirring for 2 to 3 hours, the reaction was completed. The resultant was cooled, and the temperature was controlled at -5°C to 5°C. 30.2 g of triethylamine was added dropwise. After the addition was completed, 7.04 g of water, 12 g of 5-methyl-4,5,6,7-tetrahydro-thiazolo[5,4-c]pyridine-2-carboxylic acid hydrochloride, 10.6 g of EDCI, and 7.06 g of HOBt were added successively. After the addition was completed, the temperature was heated. The mixture was maintained at 35°C to 45°C and reacted for 8 hours. After the reaction was completed, the system was distilled under reduced pressure at 35°C to 45°C to concentrate to 1/5 of its original volume. Then 400 g of 5% aqueous solution of sodium bicarbonate was added dropwise. After the addition was completed, the mixture was maintained at 5°C to 15°C and stirred for crystallisation for 1.5 to 3.0 hours. The resultant was filtered under suction. The filter cake was washed with 40 g of water and then dried under suction to obtain an off-white solid. The resultant was dried under reduced pressure at 40°C to 50°C for 10 to 12 hours to obtain an off-white solid, with a yield of 76.3%, a purity of 97.21%, and a deprotected impurity of 2.26%.

### Example 8: preparation method of edoxaban (with 0.5% water addition)

20 g of edoxaban intermediate compound 1 obtained in Example 2 was dissolved in 316 g of acetonitrile. The temperature was controlled at 15°C to 25°C. 16.4 g of methanesulfonic acid was added dropwise. After stirring for 2 to 3 hours, the reaction was completed. The resultant was cooled, and the temperature was controlled at -5°C to 5°C. 30.2 g of triethylamine was added dropwise. After the addition was completed, 1.76 g of water, 12 g of 5-methyl-4,5,6,7-tetrahydro-thiazolo[5,4-c]pyridine-2-carboxylic acid hydrochloride, 10.6 g of EDCI, and 7.06 g of HOBt were added successively. After the addition was completed, the temperature was heated. The mixture was maintained at 35°C to 45°C and reacted for 6 hours. After the reaction was completed, the system was distilled under reduced pressure at 35°C to 45°C to concentrate to 1/5 of its original volume. Then 400 g of 5% aqueous solution of sodium bicarbonate was added dropwise. After the addition was completed, the mixture was maintained at 5°C to 15°C and stirred for crystallisation for 1.5 to 3.0 hours. The resultant was filtered under suction. The filter cake was washed with 40 g of water and dried under suction to obtain an off-white solid. The resultant was dried under reduced pressure at 40°C to 50°C for 10 to 12 hours to obtain an off-white solid, with a yield of 78%, a purity of 97.24%, and a deprotected impurity of 2.32%.

### Example 9: preparation method of edoxaban (with 5% water addition)

20 g of edoxaban intermediate compound 1 obtained in Example 2 was dissolved in 316 g of acetonitrile. The temperature was controlled at 15°C to 25°C. 16.4 g of methanesulfonic acid was added dropwise. After stirring for 2 to 3 hours, the reaction was completed. The resultant was cooled, and the temperature was controlled at -5°C to 5°C. 30.2 g of triethylamine was added dropwise. After the addition was completed, 17.6 g of water, 12 g of 5-methyl-4,5,6,7-tetrahydro-thiazolo[5,4-c]pyridine-2-carboxylic acid hydrochloride, 10.6 g of EDCI, and 7.06 g of HOBt were added successively. After the addition was completed, the temperature was heated. The mixture was maintained at 35°C to 45°C and reacted for 8 hours. After the reaction was completed, the system was distilled under reduced pressure at 35°C to 45°C to concentrate to 1/5 of its original volume. Then 400 g of 5% aqueous solution of sodium bicarbonate was added dropwise. After the addition was completed, the mixture was maintained at 5°C to 15°C and stirred for crystallisation for 1.5 to 3.0 hours. The resultant was filtered under suction. The filter cake was washed with 40 g of water and then dried under suction to obtain an off-white solid. The resultant was dried under reduced pressure at 40°C to 50°C for 10 to 12 hours to obtain an off-white solid, with a yield of 78.4%, a purity of 98.7%, and a deprotected impurity of 1.19%.

**Table 1: Reaction Phenomena and Results for Synthesising Edoxaban Intermediate Compound 1 in Reference Examples 1 to 2 and Example 2**

| **No.** | **Reaction State** | **Triethylamine Equivalent** | **Intermediate Compound 1 Yield %** | **Intermediate Compound 1 Purity %** | **Total Impurity %** |
|---|---|---|---|---|---|
| Reference Example 1 | Severely Solidified, not Stirrable | 6.25eq | Post-treatment not Performed When This Phenomenon Appears in the Reaction | | |
| Reference Example 2 | Viscous, Difficult to Stir | 3.6eq | 91.7 | 97.4 | 2.6 |
| **Example 2** | **Stirrable Turbid Liquid** | 3eq | 98.20 | 99.80 | 0.2 |

**Table 2: Comparison of Experimental Results for Reference Example 1 and Examples 5-8**

| **No.** | Reaction Time | **Yield %** | **Purity %** | Deprotected Impurity | Total Impurity % |
|---|---|---|---|---|---|
| Reference Example 1 | **16 hours** | 78.9% | 95.5% | 2.93% | 4.50% |
| **Example 5** | **7 hours** | 77.6% | 98.11% | 1.52% | 1.99% |
| **Example 6** | **7 hours** | 78.3% | 98.25% | 1.32% | 1.58% |
| **Example 7** | **8 hours** | 76.3% | 97.21% | 2.26% | 2.79% |
| **Example 8** | **6 hours** | 78% | 97.24% | 2.32% | 2.76% |
| **Example 9** | **8 hours** | 78.4% | 98.70% | 1.19% | 1.30% |

The experimental results above demonstrate that the preparation method of edoxaban provided by the present application can effectively solve the problem of viscosity and solidification of the reaction system and reduce the usage amount of triethylamine. Moreover, in the step of preparing edoxaban, the addition of protic solvent can accelerate the reaction process, shorten the reaction time and improve the reaction efficiency to obtain edoxaban with high purity, and the product quality is thereby improved.

The specific examples described in the present application merely exemplify the spirit of the present application. Those skilled in the art of the present application may make various modifications or additions to the specific embodiments described or adopt similar substitutions, but will not depart from the spirit of the present invention or exceed the scope defined in the appended claims.

## Claims

1. A preparation method for edoxaban or a salt or hydrate thereof, wherein the method comprises following steps:
step 1: under acidic condition and in a polar solvent, removing a N-Boc protecting group of compound 1 to generate compound 2; 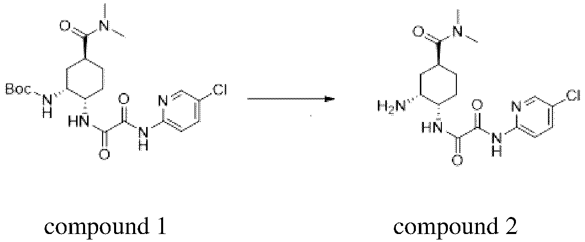
step 2: adding triethylamine, a protic solvent, a condensing agent, and 5-methyl-4,5,6,7-tetrahydro-thiazolo[5,4-c]pyridine-2-carboxylic acid or a salt thereof into a reaction solution obtained in step 1, and reacting to obtain edoxaban;

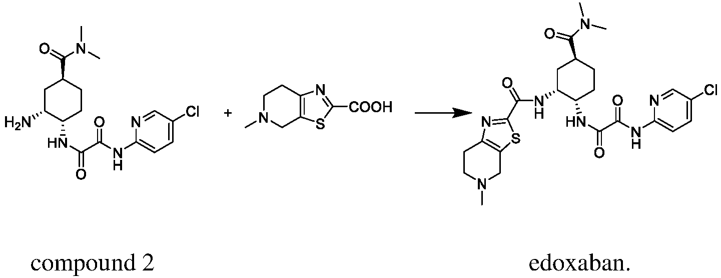

2. The preparation method according to claim 1, wherein the acidic condition in step 1 is provided by hydrochloric acid, trifluoroacetic acid, or methanesulfonic acid, preferably methanesulfonic acid; the polar solvent is selected from the group consisting of methanol, ethanol, propanol, acetonitrile, and a combination thereof, preferably acetonitrile; and the compound 1, an acid used for the acidic condition and the polar solvent are in a mass ratio of 1:0.6:15 to 1:0.9:20.

3. The preparation method according to claim 1, wherein the protic solvent in step 2 is selected from the group consisting of methanol, ethanol, water, and a combination thereof.

4. The preparation method according to claim 1, wherein the condensing agent is a combination of EDCI and HOBt.

5. The preparation method according to claim 1, wherein the compound 1, the 5-methyl-4,5,6,7-tetrahydro-thiazolo[5,4-c]pyridine-2-carboxylic acid or the salt thereof, and the triethylamine in step 2 are in a molar ratio of 1:1.1:6 to 1:1.2:8.

6. The preparation method according to claim 1, wherein the protic solvent is in a usage amount of 0.5 wt.% to 5 wt.%, preferably 3.5 wt.% to 5 wt.% of the total weight of all materials in a reaction system of step 1.

7. The preparation method according to claim 1, wherein the compound 1, EDCI, and HOBt are in a mass ratio of 1:0.4:0.3 to 1:0.6:0.5.

8. The preparation method according to claim 1, wherein the reaction solution obtained in step 1 is treated first with the triethylamine at -5°C to 5°C, and then the protic solvent, the condensing agent, and the 5-methyl-4,5,6,7-tetrahydro-thiazolo[5,4-c]pyridine-2-carboxylic acid or the salt thereof are added.

9. The preparation method according to claim 1, wherein a reaction in step 2 is at a temperature of 30°C to 50°C; and the reaction in step 2 is for a time of 6 to 8 hours.

10. The preparation method according to claim 1, wherein a post-treatment method for the edoxaban obtained in step 2 is adding dropwise an aqueous solution of sodium bicarbonate which is at a temperature of 0°C to 20°C to a reaction solution obtained in step 2, controlling temperature and stirring for crystallisation.

11. The preparation method according to claim 1, wherein the compound 1 is prepared by following steps:
step A: adding separately methyl [(5-chloropyridin-2-yl)amino]-2-oxoacetate and tert-butyl {(1R,2S,5S)-2-amino-5-[(dimethylamino)carbonyl]cyclohexyl}carbamate oxalate to a polar solvent to obtain a reaction mixture;
step B: heating the reaction mixture obtained in step A to 60 ± 5°C, then adding triethylamine in one batch and reacting to prepare compound 1;

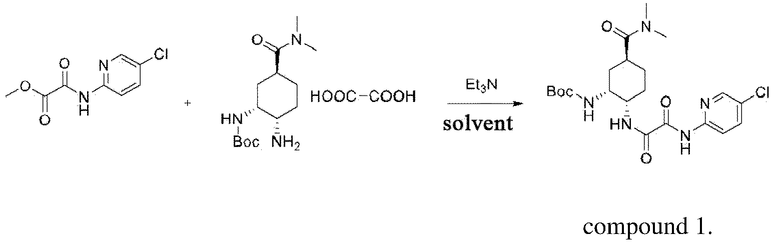

12. The preparation method according to claim 11, wherein the polar solvent is selected from the group consisting of methanol, ethanol, propanol, acetonitrile, and a combination thereof, preferably acetonitrile.

13. The preparation method according to claim 11, wherein the tert-butyl{(1R,2S,SS)-2-amino-5-[(dimethylamino)carbonyl]cyclohexyl}carbamate oxalate, the methyl [(5-chloropyridin-2-yl)amino]-2-oxoacetate, and the triethylamine in step B are in a molar ratio of 1.0:1.0:2.8 to 1.0:1.1:3.5.

14. The preparation method according to claim 11, wherein the tert-butyl{(1R,2S,5S)-2-amino-5-[(dimethylamino)carbonyl]cyclohexyl}carbamate oxalate and the polar solvent is in a mass-to-volume ratio of 1.0:4.5 to 1.0:5.5 g/mL.

15. The preparation method according to claim 11, wherein the methyl [(5-chloropyridin-2-yl)amino]-2-oxoacetate is prepared by following steps:
(1) reacting 2-amino-5-chloropyridine with methyl oxalyl chloride in tetrahydrofuran to obtain methyl [(5-chloropyridin-2-yl)amino]-2-oxoacetate hydrochloride;
(2) adding the methyl [(5-chloropyridin-2-yl)amino]-2-oxoacetate hydrochloride to tetrahydrofuran, and then adding sodium bicarbonate in batches to liberate the methyl [(5-chloropyridin-2-yl)amino]-2-oxoacetate.
